# EUROPEAN PATENT APPLICATION

(11) **EP 2 216 002 A1**
(43) Date of publication of application: **11.08.2010**
(21) Application number: 08850137.4
(22) Date of filing: 06.11.2008
(51) Int. Cl.: A61F 13/49, A61F 13/53

(54) **ABSORBENT ARTICLE**

(30) Priority: 16.11.2007 JP 2007298614
(71) Applicant: Unicharm Corporation, Ehime 799-0111 (JP)
(72) Inventor: KAWAKAMI, Yusuke, Kanonji-shi Kagawa 769-1602 (JP); SHIRAISHI, Tsukasa, Kanonji-shi Kagawa 769-1602 (JP)
(74) Representative: Peter, Julian
(86) International application number: PCT/JP2008/070228
(87) International publication number: WO 2009/063796

(57) **Abstract**

According to an absorptive article (disposable diaper) 10 of the present invention, an absorber (absorptive structure) 12 is provided with a first region 55 disposed on an internal surface sheet 41 side and having a higher content ratio of the superabsorptive polymer than a content ratio of the hydrophilic fiber, a second region 56 disposed on an external surface sheet 42 side and having a lower content ratio of the superabsorptive polymer than the content ratio of the superabsorptive polymer in the first region and a higher content ratio of the hydrophilic fiber than the content ratio of the hydrophilic fiber in the first region, and a space that penetrates from the external surface sheet 42 side of the second region 56 to the first region 55, and that can be entered by the superabsorptive polymer that is swollen by absorbing a body fluid in the first region 55.

## Description

### [Technical Field]

The present invention relates to an absorptive article used for a disposable paper diaper, a sanitary napkin, a panty liner, an incontinent pad, or the like.

### [Background Art]

In a Patent Document 1, an absorptive sheet (absorptive structure) that includes an absorber interposed between a body fluid-permeable front-surface material and a body fluid-impermeable back-surface material is disclosed as the absorptive article. The absorptive sheet includes voids formed around at least a part of superabsorptive polymer particles, and the voids have a size sufficient to accommodate the swelling of the polymer particles resulted from their increased volumes due to liquid absorption.

In this manner, the formation of voids around the polymer particles makes it possible to cause the space to accommodate the swelling of the polymer particles even if the polymer particles absorb a liquid and their volumes are increased. Therefore, so-called gel blocking of polymer particles can be effectively prevented. In particular, the presence of voids around the polymer particles leads to the formation of a coarse network by a textile material. This coarse network enables a liquid to promptly pass through the absorptive article in the thickness direction thereof. Gel blocking hardly occurs even after liquids are repeatedly absorbed. Thereby, a high passing speed of a liquid is maintained.
Patent Document: Japanese Patent Application Publication No. 2007-61171.

### [Disclosure of the Invention]

However, when voids are provided ground polymer particles within an absorber, the voids are likely formed at various positions. When the voids are not formed uniformly, gel blocking is partially generated. Hence, a body fluid cannot penetrate to the body fluid-impermeable back surface side, whereby the body fluid that has not been diffused and not been absorbed by the textile material might leak.

### [Summary of Invention]

An object of the present invention is to provide an absorptive article that can prevent the gel blocking more effectively.

In order to achieve the object of the present invention, the present invention is summarized as an absorptive article, including: an absorber that includes a superabsorptive polymer and a hydrophilic fiber, and that is interposed between a body fluid-permeable front-surface material which is permeable to a body fluid and disposed on a skin contact side and a body fluid-impermeable back-surface material which is impermeable and disposed on a clothing contact side. Here, the absorber is formed by continuously laminating a first region and a second region in a thickness direction of the absorber. The first region is disposed on a body fluid-permeable front-surface material side, and has a higher content ratio of the superabsorptive polymer and a lower content ratio of the hydrophilic fiber. The second region is disposed on a body fluid-impermeable back-surface material side, and has a lower content ratio of the superabsorptive polymer than the content ratio of the superabsorptive polymer in the first region and a higher content ratio of the hydrophilic fiber than the content ratio of the hydrophilic fiber in the first region. The absorber is provided with a groove-shaped space that penetrates from the back-surface material side of the second region to the first region, and that is extended in a planar direction of the article.

In this absorptive article, when a body fluid is discharged into the body fluid-permeable front-surface material side, the body fluid is absorbed to the hydrophilic fiber in the first region and diffuses therein, while the body fluid is also absorbed by the superabsorptive polymer in the first region. The superabsorptive polymer in the first region swells when absorbing a body fluid. The swollen superabsorptive polymer enters the space. In addition, the body fluid that passed through the first region is absorbed by the hydrophilic fiber in the second region and diffuses therein, while the body fluid is also absorbed by the superabsorptive polymer in the second region.

In such case, the superabsorptive polymer in the first region swells by absorbing a body fluid and the superabsorptive polymer enters the space. Thereby, gel blocking is suppressed without positioning the superabsorptive polymer on the body fluid-permeable front-surface material side.

Moreover, the second region has a higher content ratio of the hydrophilic fiber compared to the first region. Thus, it is possible to effectively absorb a body fluid by sufficiently exerting the capillary effect. The body fluid sufficiently diffuses in the longitudinal and transverse directions of the body fluid absorptive article.

Furthermore, the space is opened on the body fluid-impermeable back-surface material side, and penetrates from the second region to the first region. This makes it possible to uniformly form the space in a predetermined position of the absorptive structure, thereby being capable of stably suppressing gel blocking and exerting a sufficient diffusion effect, as compared with the case where voids are disposed around superabsorptive polymers.

Furthermore, in the present invention, the space is formed in a lattice shape having a plurality of longitudinal grooves. Here, the plurality of longitudinal grooves is extended along a longitudinal direction of the absorptive article and has predetermined intervals in a transverse direction of the absorptive article, and the plurality of transverse grooves is extended along the transverse direction of the absorptive article and has predetermined intervals in the longitudinal direction of the absorptive article. Intersections of the longitudinal grooves and the transverse grooves are communicated with each other.

In the absorptive article, the space is formed by the lattice shape having a plurality of longitudinal grooves and transverse grooves. Accordingly, the superabsorptive polymer in the first region, which swells by absorbing a body fluid, enters the longitudinal and transverse grooves. Thus, the swollen superabsorptive polymer is prevented from positioning on the body fluid-permeable front surface side, thereby the gel blocking can be further suppressed.

Moreover, in the invention of this application, the absorber is uniformly dispersed in the thickness direction of the superabsorptive polymer in the first region.

Furthermore, the absorber is covered with a carrier non-woven fabric on the body fluid-impermeable back surface side, and is covered with a cover non-woven fabric on the body fluid-permeable front surface side. The cover non-woven fabric has a higher ventilation resistance compared to the carrier non-woven fabric.

In the absorptive article as described above, when the absorber absorbs a body fluid, the water vapor from the body fluid absorbed in the absorber hardly leaves the cover non-woven fabric because the ventilation resistance of the cover non-woven fabric is high, whereby the water vapor leaves from the carrier non-woven fabric side where the ventilation resistance is low. As a result, the absorptive article can keep comfortableness therein.

### [Brief Description of Drawings]

[Fig. 1] Fig. 1 is a partially broken perspective view that shows a disposable diaper to which this invention is applied.
[Fig. 2] Fig. 2 is a developed plan view of the disposable diaper of Fig. 1.
[Fig. 3] Fig. 3 is a rear view in which a part of an absorber is expanded and viewed from the body fluid-impermeable back surface side.
[Fig. 4] Fig. 4 is a cross-sectional view taken along the IV-IV line of Fig. 3, in which the part of the absorber is expanded.
[Fig. 5] Fig. 5 is an explanatory view in which a part of the absorber is expanded to show an intersection of longitudinal and transverse grooves.
[Fig. 6] Fig. 6 is an explanatory view that indicates an order in which an absorber is laminated and a state in which the absorber absorbs a body fluid and swells.

### [Description of Embodiments]

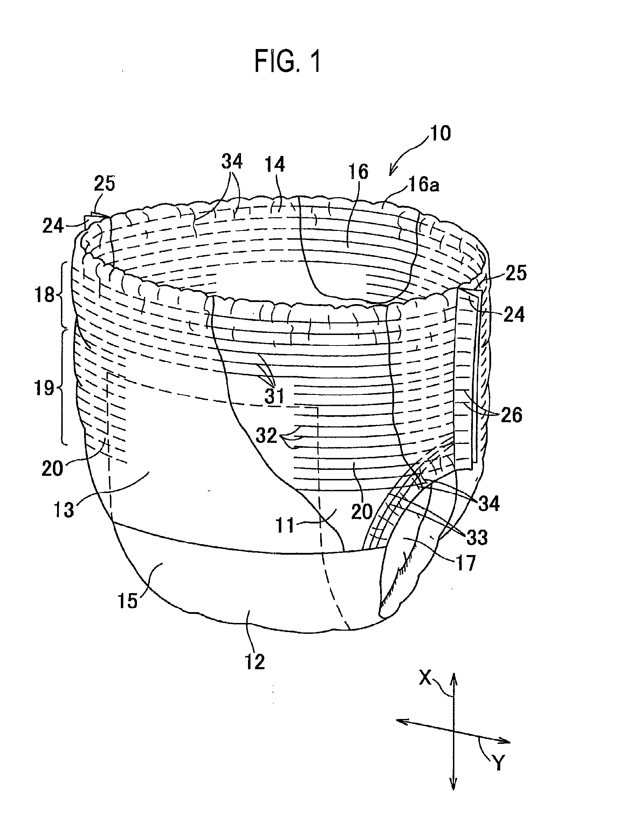

Hereinafter, an embodiment of the present invention will be described with reference to the accompanying drawings. Fig. 1 is a partially broken perspective view that shows a wearing state of a disposable diaper 10 to which a body fluid absorptive article according to the present invention is employed.

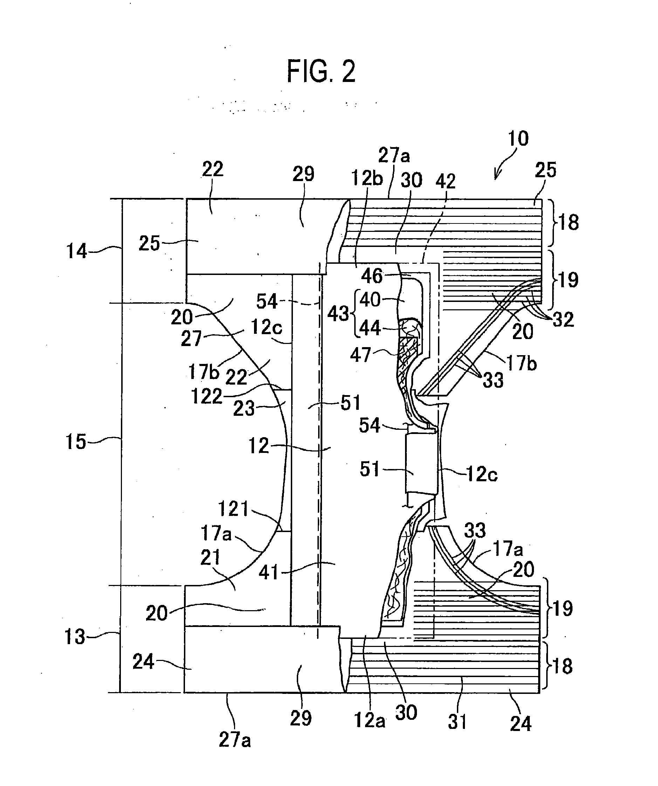

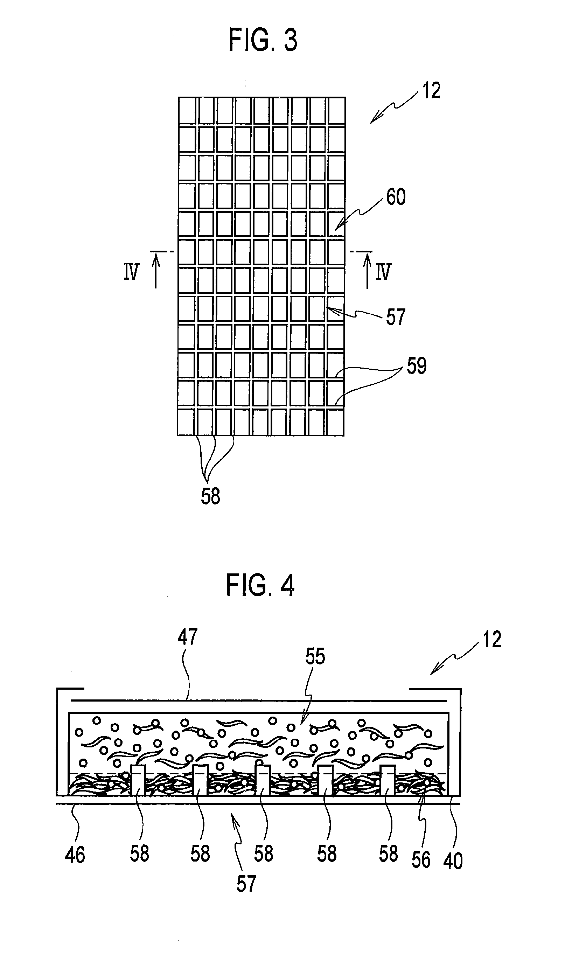

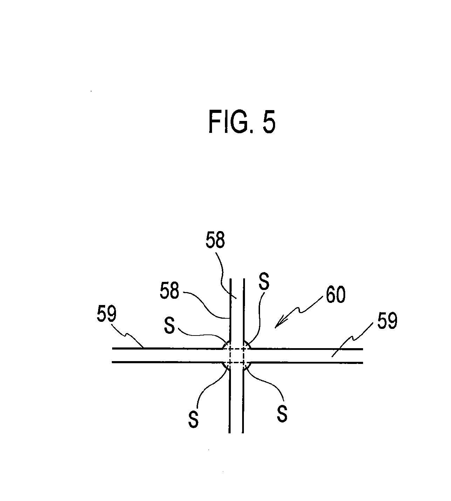

Fig. 2 is a developed plan view of the disposable diaper 10 of Fig. 1. Fig. 3 is a plan view of an absorptive structure 12 viewed from the clothing contact side. Fig. 4 is a cross-sectional view along the IV-IV line of Fig. 3. Fig. 5 is an enlarged plan view of an intersection.

A body fluid absorptive article according to the present invention is not limited to this embodiment. For instance, there can be employed sanitary napkins, panty liners, incontinent pads, and the like.

### (Configuration of disposable diaper)

The disposable diaper 10 (hereinafter referred to as, "diaper") of the present embodiment is configured of: a chassis 11 for covering a waistband portion; a liquid-permeable'absorptive structure (absorber) 12 having a liquid-absorption property, which is provided inside the chassis 11, i.e., the skin contact side of the chassis 11, and which extends in a longitudinal direction X. The diaper 10 includes a front waist region 13; a rear waist region 14; and a crotch region 15 extending therebetween. The front waist region 13 and the rear waist region 14 are provided with a plurality of first waist elastic members 31 and a plurality of second waist elastic members 32. The plurality of first waist elastic members 31 extends in a transverse direction Y along a waist opening edge 16a. The plurality of second waist elastic members 32 extends in the transverse direction Y through each of the side parts 20 of the front and rear waist regions 13, 14.

Fig. 2 is a developed plan view of the diaper 10, which is peeled off upward and downward at seam parts 26, and which is spread in the longitudinal direction X and the transverse direction Y.

The chassis 11 is configured of: a front member 21 having an almost trapezoidal shape and including the front waist region 13; a rear member 22 having an almost trapezoidal shape and including the rear waist region 14; and an intermediate member 23 having a rectangular shape and being located between the front member 21 and the rear member 22, and which forms the crotch region 15. The chassis 11 has an almost sand clock shape, as a whole, in which those front member 21, intermediate member 23 and rear member 22 are arranged in the longitudinal direction X and connected to each other in junctions 121, 122. Two side edges 24, 25 respectively of the front member 21 and the rear member 22 are overlapped with each other and connected at the seam parts 26 that line up intermittently in the longitudinal direction X, with well known joining means, e.g., a hotmelt adhesive, or by hot embossing or sonic heat-welding means to thereby define a waist opening 16 and a pair of leg openings 17 (see Fig. 1).

The front member 21, the intermediate member 23 and the rear member 22 are formed by connecting an inner layer sheet 27 located on the skin-contacting surface side to an outer layer sheet 28 located on the skin-noncontacting surface side. Here, the inner layer sheet is a liquid-permeable sheet and the outer layer sheet is a liquid-nonpermeable sheet. Both the inner layer sheet 27 and the outer layer sheet 28 are formed with an air-permeable fibrous non-woven cloth. The outer layer sheet 28 is extended from both front and rear edges 27a of the inner layer sheet 27 in the longitudinal direction X. After the absorptive structure 12 is provided inside the chassis 11, the outer layer sheet 28 is folded to the absorptive structure 12 side so as to cover the front and rear edges 12a, 12b of the absorptive structure 12 and jointed at the two side edges 24, 25. Thus, an extending part 29 of the outer layer sheet 28 covers the front and rear edges 12a, 12b of the absorptive structure 12 from the upper side. Accordingly, even when a body fluid not absorbed into the absorptive structure 12 leaks naturally from the front and rear edges 12a, 12b, the extending part 29 serves as an anti-leak barrier. Thereby, the body fluid can be prevented from leaking toward outside.

Between the inner layer sheet 27 and the outer layer sheet 28, the first waist elastic members 31, the second waist elastic members 32, and a plurality of leg elastic members 33 are provided. The leg elastic members 33 are extending along upper and lower edges 17a, 17b to be positioned around the leg. These elastic expansion and contraction members 31, 32, 33 are installed, under elongation, at least in the inner layer sheet 27 between the two sheets 27, 28 with a hotmelt adhesive (not shown). These elastic members 31, 32, 33 shrink to thereby generate a plurality of gathers 34 in the chassis 11 (see Fig. 1).

### (Configuration of absorptive structure 12)

The absorptive structure 12 forms a substantially rectangle shape as shown in Fig. 2. The absorptive structure 12 includes: a internal surface sheet 41 that is liquid-permeable; an external surface sheet 42 that is liquid-nonpermeable; and a core material 43 that is liquid-absorptive and is interposed between the internal surface sheet 41 and the external surface sheet 42. In this embodiment, the internal surface sheet 41 and the external surface sheet 42 extend from the circumference of the core material 43 that is formed into substantially a rectangular shape, and overlap with and are jointed to each other by use of a hotmelt adhesive (not shown). As a result, formed are: the front edge 12a facing a front-end edge 21 a of the chassis 11; the rear edge 12b facing a rear-end edge 22a of the chassis 11; and two side edges 12c that extend in the longitudinal direction X between the front and rear edges 12a, 12b. The front and rear edges 12a, 12b are respectively connected to the inner layer sheet 27 of the front member 21 and the inner layer sheet 27 of the rear member 22 by use of a hotmelt adhesive (not shown).

The core material 43 is formed by covering an absorptive core 44 with a carrier non-woven fabric 40. The absorptive core 44 is made from a mixture of a flap pulp, a highly absorptive absorber and, as required, a heat-welding staple fiber, or the like. A liquid barrier sheet 46 formed of a liquid-nonpermeable, moisture-permeable plastic film is provided between the carrier non-woven fabric 40 and the external surface sheet 42. The liquid barrier sheet 46 is only required to be large enough to be positioned in the bottom of the core material 43. However, the barrier sheet 46 preferably has a size to cover the entire bottom of the core material 43 in order tc exert a sufficient leakproof effect. Moreover, a liquid-permeable cover non-woven fabric 47 is provided between the core material 43 and the internal surface sheet 41.

A pair of barrier cuffs 51, which extend in the longitudinal direction X, and which are formed from a liquid-nonpermeable sheet, are installed in the respective side edges 12c of the absorptive structure 12.

In the absorptive structure 12 of this embodiment, as shown in Fig. 4, a first region 55 and a second region 56 are continuously laminated in the thickness direction of the diaper 10. The first region 55 is disposed on the internal surface sheet 41 (body fluid-permeable front-surface material) side, and has a higher content ratio of a superabsorptive polymer (SAP) compared to a content ratio of a hydrophilic fiber (pulpwood). The second region 56 is disposed on the external surface sheet 42 (body fluid-impermeable back-surface material) side, and has a lower content ratio of a superabsorptive polymer compared to the first region 55 and a higher content ratio of a hydrophilic fiber compared to the first region 55. Moreover, this first region 55 contains the superabsorptive polymer that uniformly disperses in the thickness direction. In addition, a space 57 is formed by opening the second region 56 on the liquid barrier sheet 46 side, penetrating from the second region 56 to the first region 55. The superabsorptive polymer that absorbs a body fluid and swells in the first region 55 can enter the space 57.

In this absorptive structure 12, the ratios of SAP and pulp are set at 50 to 70 wt% and at 30 to 50 wt%, respectively. The weight ratio of the SAP is set larger. The target thickness of the absorptive structure 12 is set at 3.0 mm or less.

Further, the space 57 is formed in a lattice shape having a plurality of longitudinal grooves 58 and a plurality of transverse grooves 59. The plurality of longitudinal grooves 58 are extended along the longitudinal direction of the disposable diaper 10, and formed by having predetermined intervals in the transverse direction of the disposable diaper 10. The plurality of transverse grooves 59 are extended along the transverse direction of the disposable diaper 10 and formed by having predetermined intervals in the longitudinal direction of the disposable diaper 10.

As shown in Fig. 5, an intersection 60 is formed by intersecting each of the longitudinal grooves 58 and each of the transverse grooves 59 which are in a communicated state to each other. In this case, four arc parts S are formed in each of the corners made by walls 58a forming the longitudinal grooves 58 and walls 59a forming the transverse grooves 59, in the manufacturing process.
As a result, the space (volume) of the intersection 60 can be largely secured. Accordingly, the space capable of accommodating a swollen volume of the first region 55 can be largely secured, and the intersection 60 is hardly occupied.

Moreover, the absorptive structure 12 is sandwiched between the cover non-woven fabric 47 and the carrier non-woven fabric 40. The cover non-woven fabric 47 has a higher ventilation resistance compared to the carrier non-woven fabric 40.

### (Method of manufacturing absorptive structure 12).

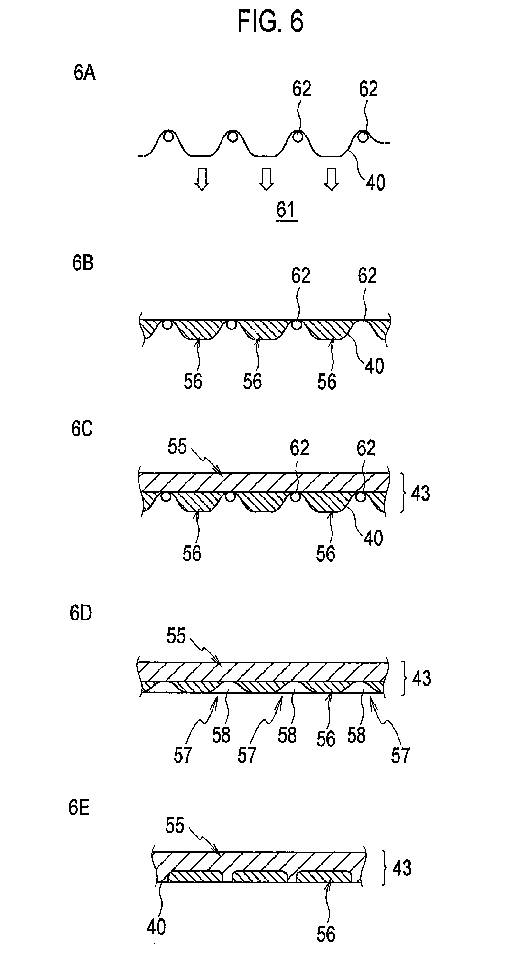

The absorptive structure 12 of the present invention can be manufactured, for example, by a known rotary drum-type laminating device. Steps of forming the first region 55 and the second region 56 using such a laminating device will be described with reference to Fig. 6.

As shown in Fig. 6(a), the carrier non-woven fabric 40 on a mesh wire net 62 disposed along the outer peripheral of the above-mentioned drum device is sucked to the internal side of a laminating drum 61 by the sucking force of the laminating drum 61. In such conditions, pulp and SAP are sprayed onto the carrier non-woven fabric 40 by a funnel. At this time, the pulp is a material lighter than SAP, so that pulp more easily arrives on the carrier non-woven fabric 40 on the wire net 62 if they are blown with the same speed of wind. As shown in Fig. 6(b), the second region 56 having a high content ratio of the pulp and has a low content ratio of the SAP is formed on the carrier non-woven fabric 40.

In addition, as shown in Fig. 6(c), the pulp and the SAP are sprayed onto the carrier non-woven fabric 40 by a funnel. Thus, the first region 55 having a high content ratio of the SAP and has a low content ratio of pulp is formed on the second region 56. Additionally, it is preferable that the sucking force of the laminating drum 61 is adjusted such that the superabsorptive polymer contained therein is uniformly dispersed in the thickness direction in the same areas in this first region 55. This is because such a uniformly dispersed superabsorptive polymer contributes to the prevention of gel blocking in the area.

Further, when the core material 43 in which the first region 55 and the second region 56 are formed is transported from the laminating drum 61, as shown in Fig. 6(d), the opening is formed in the second region 56 of the external surface sheet 42 side, and the space 57 penetrating from the second region 56 to the first region 55 is formed in the second region 56. The space 57 is formed in a shape of the mesh wire net 62. In other words, the space 57 is formed in a lattice shape having the plurality of longitudinal grooves 58 intersecting the plurality of traverse grooves 59. Here, the plurality of longitudinal grooves 58 is extended along the longitudinal direction of the absorptive structure 12 and has predetermined intervals in the transverse direction of the absorptive structure 12. Further, the plurality of transverse grooves 59 is extended along the transverse direction of the absorptive structure 12 and has predetermined intervals in the longitudinal direction of the absorptive structure 12. Note that, such formation of the space 57 penetrating the second region 56 and the first region 55 can be achieved by adjusting the sucking force of the laminating drum 61, or a similar adjustment process as appropriate.

Note that, in Fig. 6(c) and Fig. 6(d), the first region 55 and the second region 56 are divided by using a dotted line or a solid line. However, though the boundary between the first region 55 and the second region 56 formed by the above-described manufacturing method does not necessarily become a straight line and sometimes possibly becomes a curved line, the boundary is indicated with a straight line in this embodiment for the description purpose.

In addition, as illustrated in Fig. 3 and Fig. 4, the mesh wire net 62 is linearly intersected and depicted as a lattice-shaped form. In the planar direction or the thickness direction, the wire net 62 is acceptable even if it is not linear or its width is not uniform. In such a case, the entire volume of the space 57 formed in the second region 56 can be increased.

### (Operation of absorptive structure 12)

Next, the operation of the absorptive structure 12 will be described. When a body fluid is discharged into an internal surface sheet 41, the body fluid is absorbed to the hydrophilic fiber and diffuses in the first region 55 of the absorptive structure 12, and is also absorbed by a superabsorptive polymer in the first region 55. When the superabsorptive polymer in the first region 55 absorbs a body fluid, the body fluid is enclosed and swollen in a three-dimensional network of the polymer.

In this case, in the absorptive structure 12 of this embodiment, as shown in Fig. 6(e), the swollen superabsorptive polymer enters the space 57 disposed in the second region 56. In other words, the superabsorptive polymer enters the longitudinal grooves 58 and the transverse grooves 59, which are disposed in the second region 56, opened on the external surface sheet 42 side and are penetrated to the first region 55. Then, the superabsorptive polymer is swollen in the thickness direction of the absorptive structure 12. Additionally, since the longitudinal groove 58 and the transverse groove 59 are mutually communicated at the intersection 60, the volume of the intersection 60 is larger by the volume of the arc part S of the intersection 60 compared to the space constituted by only the longitudinal groove 58 or by only the transverse groove 59. Also within this large intersection 60, the superabsorptive polymer of the first region 55 is swollen in the thickness direction of the absorptive structure 12.

In addition, the body fluid that has passed through the first region 55 is absorbed by the hydrophilic fiber in the second region 56, and sufficiently diffuses in the transverse and longitudinal directions of the absorptive structure 12, while the body fluid is also absorbed by the superabsorptive polymer in the second region 56.

In this case, even when the superabsorptive polymer in the first region 55 absorbs the body fluid and swells, the superabsorptive polymer enters the space 57 and swells in the thickness direction, whereby the ratio of the number of the swollen superabsorptive polymers that are planarly positioned is small. Thus, the body fluid is readily penetrated, and gel blocking can be suppressed. In addition, the body fluid permeability can be maintained even when the body fluids are repeatedly discharged. The pulp in the second region 56 can provide a sufficient diffusion effect.

Moreover, the second region 56 has a higher content ratio of the hydrophilic fiber compared to the first region 55. Thus, it is possible to effectively absorb the body fluid by sufficiently exerting the capillary effect. Thereby, the body fluid sufficiently diffuses in the longitudinal and transverse directions of the fluid absorptive structure 12.

Furthermore, the space 57 is opened on the external surface sheet 42 side and penetrates from the second region 56 to the first region 55. This makes it possible to uniformly form the space 57 in a predetermined position of the absorptive structure 12, thereby being capable of stably suppressing gel blocking and exerting a sufficient diffusion effect, as compared with the case where voids are disposed around superabsorptive polymers.

In addition, in this embodiment, the space 57 is formed in a lattice shape having a plurality of longitudinal grooves 58 intersecting the plurality of transverse grooves 59. Further, the plurality of longitudinal grooves 58 is extended along the longitudinal direction of the absorptive structure 12 and has predetermined intervals in the transverse direction of the absorptive structure 12 and the plurality of transverse grooves 59 is extended along the transverse direction of the absorptive structure 12 and has predetermined intervals in the longitudinal direction of the absorptive structure 12. Thus, the superabsorptive polymer, which has absorbed a body fluid and swollen, in the first region 55 enters the longitudinal groove 58 and the transverse groove 59. Hence, the swollen superabsorptive polymer is prevented from positioning on the body fluid-permeable surface side, and thereby gel blocking is further suppressed.

In addition, in this embodiment, the absorptive structure 12 is covered with the internal surface sheet 41 and the external surface sheet 42, and the internal surface sheet 41 has the higher ventilation resistance compared to the external surface sheet 42. Therefore, when the absorptive structure 12 absorbs a body fluid, the water vapor from the body fluid absorbed in the absorptive structure 12 hardly leaves from the internal surface sheet 41. This is because the ventilation resistance of the internal surface sheet 41 is high. Accordingly, the water vapor leaves from the external surface sheet 42 side where the ventilation resistance is low. As a result, the absorptive article can keep comfortableness therein.

The description has been given of the embodiment to which the present invention has been applied. However, it should be noted that the present invention is not limited by the description and the drawings which are the part of the disclosure of the invention by this embodiment. Other embodiments, examples, and management techniques, etc, which are performed by those skilled in the art, and the like, on the basis of the above embodiment, are all included in the scope of the present invention.

The entire contents of the Japanese Patent Application No. JP 2007-298614 (filed on November 16, 2007) are incorporated herein by reference.

### [Industrial Applicability]

As mentioned above, the absorptive article of the present invention, even though the superabsorptive polymer in the first region swells by absorbing a body fluid, the superabsorptive polymer enters the space formed in the second region and swells mainly in the thickness direction of the absorber. Accordingly, it is possible to prevent the superabsorptive polymer from to greatly extending in the planar direction, and the gel blocking can be suppressed. Therefore, even if the body fluid is repeatedly discharged, the body fluid permeability can be maintained as much as possible. The hydrophilic fiber in the second region can provide a sufficient diffusion effect.

Moreover, the second region has a lower content ratio of the hydrophilic fiber compared to the first region. Accordingly, the occupancy of the space due to the swelling of the superabsorptive polymer in the second region can be avoided as much as possible. The gel blocking in the vicinity of the boundary between the first region and second region can be prevented particularly effectively. In addition, since the ratio of the fibrous material is high in the second region, the capillary action enables the body fluid to move in the planar direction. Thus, the possibility of concentrated gel blocking is also suppressed.

Additionally, although the space is configured to penetrate from a surface of the second region to the first region, the space can be uniformly formed in a predetermined position of the absorber, because forming a groove-shaped space in a surface layer is relatively easy. Thereby, it is possible to stably suppress the gel blocking and obtain a sufficient diffusion effect, as compared with the case where voids are disposed around superabsorptive polymers.

## Claims

1. An absorptive article, comprising:
an absorber that includes a superabsorptive polymer and a hydrophilic fiber,
and that is interposed between a body fluid-permeable front-surface material disposed on a skin contact side and a body fluid-impermeable back-surface material disposed on a clothing contact side, wherein
the absorber is formed by continuously laminating a first region and a second region in a thickness direction of the absorber,
the first region is disposed on a body fluid-permeable front-surface material side,
and has a higher content ratio of the superabsorptive polymer than a content ratio of the hydrophilic fiber,
the second region is disposed on a body fluid-impermeable back-surface material side, and has a lower content ratio of the superabsorptive polymer than the content ratio of the superabsorptive polymer in the first region and a higher content ratio of the hydrophilic fiber than the content ratio of the hydrophilic fiber in the first region, and
the absorber is provided with a groove-shaped space that penetrates from the back-surface material side of the second region to the first region, and that is extended in a planar direction of the article.

2. An absorptive article, comprising:
an absorber that includes a superabsorptive polymer and a hydrophilic fiber, and that is interposed between a body fluid-permeable front-surface material disposed on a skin contact side and a body fluid-impermeable back-surface material disposed on a clothing contact side, wherein
the absorber is formed by continuously laminating a first region and a second region in a thickness direction of the absorber,
the first region is disposed on a body fluid-permeable front-surface material side,
and has a higher content ratio of the superabsorptive polymer than a content ratio of the hydrophilic fiber,
the second region is disposed on a body fluid-impermeable back-surface material side, and has a lower content ratio of the superabsorptive polymer than the content ratio of the superabsorptive polymer in the first region and a higher content ratio of the hydrophilic fiber than the content ratio of the hydrophilic fiber in the first region, and
the absorber is provided with a space that penetrates from the back-surface material side of the second region to the first region, and that can be entered by the superabsorptive polymer that is swollen by absorbing a body fluid in the first region.

3. The absorptive article according to any one of claims 1 and 2, wherein the space is formed in a lattice shape having a plurality of longitudinal grooves intersecting a plurality of transverse grooves,
the plurality of longitudinal grooves is extended along a longitudinal direction of the absorptive article and has predetermined intervals in a transverse direction of the absorptive article, and
the plurality of transverse grooves is extended along the transverse direction of the absorptive article and has predetermined intervals in the longitudinal direction of the absorptive article.

4. The absorptive article according to claim 3, wherein
in the first region, the superabsorptive polymer is uniformly dispersed in the thickness direction of the absorber.

5. The absorptive article according to any one of claims 1 to 4, wherein the absorber is covered with a carrier non-woven fabric on the body fluid-impermeable back-surface material side, and is covered with a cover non-woven fabric on the body fluid-permeable front-surface material side, and the cover non-woven fabric has a higher ventilation resistance compared to the carrier non-woven fabric.

6. An absorber, comprising:
a superabsorptive polymer; and
a hydrophilic fiber, wherein
the absorber is formed by continuously laminating a first region and a second region in a thickness direction of the absorber,
the first region has a high content ratio of the superabsorptive polymer and a low content ratio of the hydrophilic fiber,
the second region has a lower content ratio of the superabsorptive polymer and
a higher content ratio of the hydrophilic fiber compared to the first region, and
the absorber is provided with a space that penetrates from a surface layer of the second region to the first region, and that can be entered by the superabsorptive polymer swollen by absorbing a body fluid in the first region.
